# EUROPÄISCHE PATENTANMELDUNG

(11) **EP 0 752 411 A2**
(43) Veröffentlichungstag der Anmeldung: **08.01.1997**
(21) Anmeldenummer: 96110198.7
(22) Anmeldetag: 25.06.1996
(51) Int. Cl.: C07C 209/60

(54) **Verfahren zur Herstellung von Aminen aus Olefinen an Pillared Clays**

(30) Priorität: 04.07.1995 DE 19524242
(71) Anmelder: BASF AKTIENGESELLSCHAFT, 67056 Ludwigshafen (DE)
(72) Erfinder: Dingerdissen, Uwe, Dr., 64342 Seeheim-Jugenheim (DE); Eller, Karsten, Dr., 67059 Ludwigshafen (DE)

(57) **Zusammenfassung**

Verfahren zur Herstellung von Aminen der allgemeinen Formel I 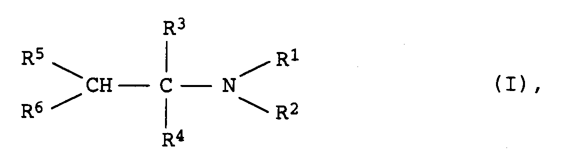 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam eine gesättigte oder ungesättigte C₃- bis C₉-Alkylendikette und
- R³ oder R⁵: C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 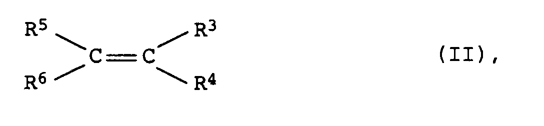 in der R³, R⁴, R⁵ und R⁶ die oben genannten Bedeutungen haben, mit Ammoniak oder primären oder sekundären Aminen der allgemeinen Formel III 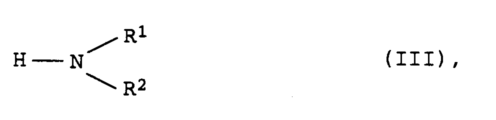 in der R¹ und R² die oben genannten Bedeutungen haben, bei Temperaturen von 200 bis 350°C und Drücken von 100 bis 300 bar in Gegenwart eines Heterogenkatalysators, indem man als Heterogenkatalysator Pillared Clays einsetzt.

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drücken in Gegenwart von Pillared Clays.

Eine Übersicht über die Methoden zur Aminierung von Olefinen wird in "Functionalisation of Alkenes: Catalytic Amination of Monoolefins", J.J. Brunet et al. J.Mol.Catal., 49 (1989), Seiten 235 bis 259 gegeben.

Grundsätzlich gibt es zwei Katalysemechanismen. Das Olefin wird über ein Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus, kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 375 002, US-A-4 536 602, EP-A-305 564, EP-A-101 921, DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder säuremodifizierte Beta-Zeolithe eingesetzt.

Ein besonderer Nachteil der Zeolithe als Katalysatoren liegt in ihrer aufwendigen Herstellung und damit in ihrem hohem Preis.

Die selektive Synthese eines Molekularsiebs (z.B von Zeolithen) durch Hydrothermalsynthese erfordert die genaue Einhaltung vieler Parameter, wie etwa der Kristallisationszeit, der Kristallisationstemperatur, des Druckes oder der Alterungsschritte.

Die bei einer Zeolithsynthese üblicherweise eingesetzte strukturdirigierende Verbindung (Templat) muß nach der Kristallisation entfernt werden. Die Entfernung des Templats geschieht in der Regel durch Calcinierung, wobei die organische Verbindung oxidativ abgebaut wird. Aus ökologischen und ökonomischen Gründen ist dies sehr negativ zu bewerten.

Die Herstellung von Kristallen bestimmter Größe oder Morphologie sowie die Präparation geträgerter Zeolithe, welche als Katalysatoren aufgrund verfahrenstechnischer Vorteile oft wünschenswert wäre, ist in der Regel nicht oder nur mit viel Aufwand möglich.

Zeolithe besitzen eine sehr enge Porengrößenverteilung. Die Porengrößen variieren je nach Zeolithtyp von 4 bis ca. 12 Å.

Bei einer Zeolith-katalysierten Reaktion haben nur solche Moleküle Zugang zu den katalytisch aktiven Zentren im Inneren des Zeoliths, welche kleiner sind als die Porendimensionen. Reaktanden mit größeren Abmessungen sind vom Inneren der Poren ausgeschlossen.

Für die oben genannte Aminierungsreaktionen bedeutet dies, daß die katalytischen Zentren im Inneren des Zeolithen zur Herstellung von Aminen, welche größer sind als der Porendurchmesser, nicht zur Verfügung stehen.

Der vorliegenden Erfindung lag daher die Aufgabe zugrunde, den zuvor genannten Nachteilen abzuhelfen, insbesondere Katalysatoren für die Aminierung von Olefinen zu finden, deren Präparation deutlich einfacher ist als die der Zeolith-Katalysatoren und eine günstige Porengrößenverteilung auch für voluminösere Aminmoleküle haben.

Der Einsatz von säuremodifizierten Montmorilloniten wird in EP-469719 beschrieben. Die Verwendung von Fällkatalysatoren unter Kombination von zwei oder mehreren Metalloxiden ausgenommen der Kombination Si und Al wird in JP-04082864 dargestellt.

Alle Verfahren zur Synthese von Aminen aus Olefinen an nichtzeolithischen Katalysatoren zeichnen sich durch eine geringe Amin-Ausbeute oder geringe Raum-Zeit-Ausbeute aus, oder führen zu einer raschen Desaktivierung der Katalysatoren.

Der vorliegenden Erfindung lag daher die Aufgabe zugrunde, diesen Nachteilen abzuhelfen.

Demgemäß wurde ein neues und verbessertes Verfahren zur Herstellung von Aminen der allgemeinen Formel I 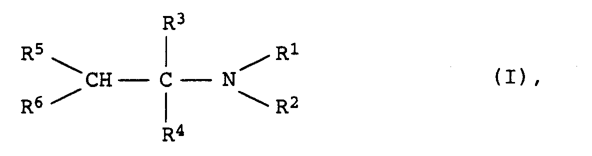 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam eine gesättigte oder ungesättigte C₃- bis C₉-Alkylendikette und
- R³ oder R⁵: C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 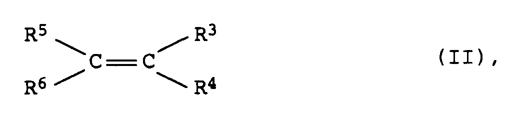 in der R³, R⁴, R⁵ und R⁶ die oben genannten Bedeutungen haben, mit Ammoniak oder primären oder sekundären Aminen der allgemeinen Formel III 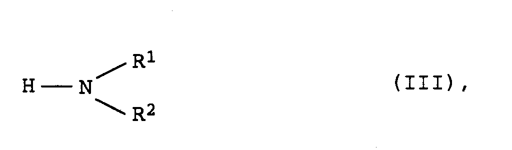 in der R¹ und R² die oben genannten Bedeutungen haben, bei Temperaturen von 200 bis 350°C und Drücken von 100 bis 300 bar in Gegenwart eines Heterogenkatalysators gefunden, welches dadurch gekennzeichnet ist, daß man als Heterogenkatalysator Pillared Clays einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart eines Pillared Clays als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich Pillared (Interlayered) Clays, die beispielsweise aus Catal. Rev. Sci. Eng., 30(3), 457 bis 499 (1988) bekannt sind.

Pillared Clays (PILCs) sind aufgebaut aus Schichten wie beispielsweise Montmorillonit, Beidellit, Hectorit oder Saponit, zwischen denen Oxide in Form von Pfeilern eingelagert sind. Die Pfeiler können z.B. durch Ionenaustausch der Schichtverbindung mit voluminösen Kationen wie [Al₁₃O₄(OH)₂₄(H₂O)₁₂]⁷⁺ oder [Zr₄(OH)₈(H₂O)₁₆]⁸⁺ gefolgt von einer Kalzinierung erhalten werden. Letztere führt zur Bildung von Al₂O₃- oder ZrO₂-Pfeilern für die genannten Kationen. Andere Oxide, die eingelagert werden können sind beispielsweise TiO₂, Cr₂O₃, SiO₂, Ta₂O₅, Fe₂O₃, Mischungen daraus oder Mischungen mit anderen Metalloxiden wie MgO. Auch Sulfide wie Fe₂S₃ können anstatt der Oxide als Pfeiler dienen. Der durch die Pfeiler zwischen den Schichtstrukturen aufgespannte Raum steht als Porenvolumen für die Reaktanden zur Verfügung. Zusätzliches Porenvolumen wird durch Delaminierung geschaffen.

Die Pillared Clays können als solche verformt werden, oder aber auch mit einem Bindemittel im Verhältnis 98:2 bis 40:60 Gew.-% zu Strängen oder Tabletten. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 h getrocknet und bei 200°C bis 500°C/2 bis 16 h calciniert, wobei die Calcinierung auch direkt im Aminierungsreaktor erfolgen kann.

Zur Erhöhung der Selektivität, der Standzeit und der Anzahl der möglichen Regenerierungen kann man verschiedene Modifizierungen an Pillared Clay-Katalysatoren vornehmen.

Eine Modifizierung der Katalysatoren besteht darin, daß man die unverformten oder die verformten Pillared Clays mit Alkalimetallen wie Na und K, Erdalkalimetallen wie Ca, Mg, Erdmetallen wie Tl, Übergangsmetallen wie z.B. Ti, Zr, Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten Pillared Clays in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium- und Alkaliform der Pillared Clays vorgenommen werden.

Eine weitere Möglichkeit der Metallaufbringung auf die Pillared Clays besteht darin, daß man das Material z.B. mit einem Halogenid, einem Nitrat, einem Acetat, einem Oxalat, einem Citrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Pillared Clays kann eine Nachbehandlung mit Wasserstoff und/oder mit Wasserdampf günstig sein.

Eine weitere Möglichkeit der Modifizierung besteht darin, daß man die Pillared Clays - verformt oder unverformt - einer Behandlung mit Säuren, wie Salzsäure (HCl), Flußsäure (HF), Schwefelsäure (H₂SO₄), Phosphorsäure (H₃PO₄) oder Oxalsäure (HO₂C-CO₂H) unterwirft.

Eine besondere Ausführungsform besteht darin, daß man die Pillared Clays vor der Verformung mit einer der genannten Säuren 0,001 n bis 2 n, bevorzugt 0,05 bis 0,5 n 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der Pillared Clays nach ihrer Verformung mit Bindemittel. Hierbei wird der Pillared Clay in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert.

Eine andere Möglichkeit der Modifizierung ist gegeben durch einen Austausch mit Ammonsalzen, z.B. mit NH₄Cl oder mit Mono-, Di- oder Polyaminen. Hierbei wird der mit Bindemittel verformte Pillared Clay in der Regel bei 60 bis 80°C mit 10 bis 25%iger, bevorzugt 20%iger NH₄Cl-Lösung 2 h kontinuierlich in gewichtsmäßiger Pillared Clay/Ammonchlorid-Lösung von 1:15 ausgetauscht und danach bei 100 bis 120°C getrocknet.

Die Katalysatoren kann man als Stränge mit Durchmessern von z.B. 1 bis 4 mm oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹,R²,R³,R⁴,R⁵,R⁶
- Wasserstoff,
- C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
- C₂- bis C₂₀-Alkenyl, bevorzugt C₂- bis C₁₂-Alkenyl, besonders bevorzugt C₂- bis C₈-Alkenyl wie Vinyl und Allyl,
- C₂- bis C₂₀-Alkinyl, bevorzugt C₂- bis C₈-Alkinyl, insbesondere C₂H und Propargyl,
- C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
- C₄- bis C₂₀-Alkyl-cycloalkyl, bevorzugt C₄- bis C₁₂-Alkyl-cycloalkyl, besonders bevorzugt C₅- bis C₁₀-Alkyl-cycloalkyl,
- C₄- bis C₂₀-Cycloalkyl-alkyl, bevorzugt C₄- bis C₁₂-Cycloalkyl-alkyl, besonders bevorzugt C₅- bis C₁₀-Cycloalkyl-alkyl,
- Aryl wie Phenyl, 1-Naphthyl und 2-Naphthyl, bevorzugt Phenyl,
- C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
- C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl, 2-Phenylethyl,
R¹ und R²
- gemeinsam eine gesättigte oder ungesättigte C₃- bis C₉-Alkylendikette, bevorzugt -(CH₂)₄-, -(CH₂)₅-, -(CH₂)₇- und -CH=CH-CH=CH-,
R³ oder R⁵
- C₂₁- bis C₂₀₀-Alkyl, bevorzugt C₄₀- bis C₂₀₀-Alkyl, wie Polybutyl, Polyisobutyl, Polypropyl, Polyisopropyl und Polyethyl, besonders bevorzugt Polybutyl und Polyisobutyl,
- C₂₁- bis C₂₀₀-Alkenyl, bevorzugt C₄₀- bis C₂₀₀-Alkenyl, besonders bevorzugt C₇₀- bis C₁₇₀-Alkenyl,
R³ und R⁵
- gemeinsam eine C₂- bis C₁₂-Alkylendikette, bevorzugt eine C₃- bis C₈-Alkylendikette, besonders bevorzugt -(CH₂)₃-, -(CH₂)₄-, -(CH₂)₅-, -(CH₂)₆- und -(CH₂)₇-, insbesondere -(CH₂)₃- und -(CH₂)₄-.

### Beispiele

### Katalysatorsynthese

### Katalysator A

142,5g AlCl₃*6H₂O wurden in 2,9l dest. Wasser und 50g NaOH in 5,5l Wasser gelöst, zueinander gegeben und 1h bei 50°C gerührt und anschließend eine Aufschlämmung von 100g Terrana D (Tonerde, Fa. Südchemie) in 4l Wasser zugefügt. Mit 21g 25% NH₄OH-Lösung wurde der pH-Wert auf 5.5 eingestellt. Die Reaktionsmischung wurde 3h bei 80°C gerührt, abfiltriert, mit dest. Wasser gewaschen, bei 100°C 2h getrocknet und bei 200°C in Luft kalziniert.

### Katalysator B

115,7g Katalysator A wurde mit 77,1g Boehmit und 3,9g Ameisensäure versetzt. Im Kneter wurde die Mischung kompaktiert und unter vorsichtigem Wasserzusatz (100 ml) verknetet. In einer Strangpresse wurden mit einem Preßdruck von 50 bar 4mm Stränge erzeugt, 16 h bei 120°C getrocknet und anschließend 5 h bei 400°C kalziniert.

Der Katalysator hatte eine BET-Oberfläche von 216 m²/g und zeigte im Röntgendiffraktogramm einen 001-Reflex bei ca. 18,4 Å.

### Katalysator C

315g Al(NO₃)₃*9 H₂O wurden in 3,9l dest. Wasser und 70g NaOH in 7,6l Wasser gelöst, zueinander gegeben und anschließend eine Aufschlämmung von 140g Terrana D (Tonerde, Fa. Südchemie) in 1,4l Wasser zugefügt. Mit 32,5g 25% NH₄OH-Lösung wurde der pH-Wert auf 5.5 eingestellt. Die Reaktionsmischung wurde 3h bei 80°C gerührt, abfiltriert, mit dest. Wasser gewaschen, bei 100°C 2h getrocknet und 5h bei 200°C in Luft kalziniert.

### Katalysator D

155,9 g Katalysator C wurde mit 103,9g Boehmit und 5,2 g Ameisensäure versetzt. Im Kneter wurde die Mischung kompaktiert und unter vorsichtigem Wasserzusatz (138 ml) verknetet. In einer Strangpresse wurden mit einem Preßdruck von 55 bar 4mm Stränge erzeugt, 16 h bei 120°C getrocknet und anschließend 5 h bei 300°C kalziniert.

Der Katalysator hatte eine BET-Oberfläche von 167 m²/g.

### Katalysator E

Zu 6l 1M HCl wurden tropfenweise 426,6g Titantetraisopropylat zugegeben, 3h bei 20 bis 25°C gerührt und mit einer Aufschlämmung von 120g Terrana D (Tonerde, Fa. Südchemie) in 12l Wasser:Aceton (1:1) unter Rühren vereint. Die Mischung wurde 3h bei 20 bis 25°C nachgerührt, abfiltriert, mit dest. Wasser gewaschen, bei 100°C 2h getrocknet und 3h bei 300°C in Luft kalziniert.

### Katalysator F

125 g Katalysator E wurde mit 31,3g Boehmit und 3,2g Ameisensäure versetzt. Im Kneter wurde die Mischung kompaktiert und unter vorsichtigem Wasserzusatz (75 ml) verknetet. In einer Strangpresse wurden mit einem Preßdruck von 60 bar 4mm Stränge erzeugt, 16 h bei 110°C getrocknet und 12 h bei 300°C kalziniert.

BET: 227 m²/g

### Katalysator G

Zu 61 ml 1M HCl wurden tropfenweise 426,6g Titantetraisopropylat zugegeben. Die Mischung wurde 3h bei 20 bis 25°C gerührt und mit einer Aufschlämmung von 120g K10 (behandelte Tonerde, Fa. Südchemie) in 12l 1M HCl:Aceton (1:1) unter Rühren vereint. Die Mischung wurde 3h bei 20 bis 25°C nachgerührt, abfiltriert, mit dest. Wasser gewaschen, bei 100°C 2h getrocknet und 3h bei 300°C in Luft kalziniert.

### Katalysator H

110,7 g Katalysator G wurde mit 27,7g Boehmit und 2,8g Ameisensäure versetzt. Im Kneter wurde die Mischung kompaktiert und unter vorsichtigem Wasserzusatz (87 ml) verknetet. Die Knetzeit betrug 60 min. In einer Strangpresse wurden mit einem Preßdruck von 45 bar 4mm Stränge erzeugt, 12 h bei 120°C getrocknet und anschließend 12 h bei 500°C kalziniert.

BET: 229 m²/g

### Katalysator I

96,8g Zirkonoxychlorid wurden in 2900g dest. Wasser gelöst und mit einer Aufschlämmung von 120g K10 (behandelte Tonerde, Fa. Südchemie) in 12l Wasser unter Rühren vereint. Die Mischung wurde 24h bei 20 bis 25°C nachgerührt, abfiltriert, mit dest. Wasser gewaschen, bei 120°C 8h getrocknet und 12h bei 200°C in Luft kalziniert.

### Katalysator J

117,9 g Katalysator I wurde mit 29,5g Boehmit und 3g Ameisensäure versetzt. Im Kneter wurde die Mischung kompaktiert und unter vorsichtigem Wasserzusatz (87 ml) verknetet. In einer Strangpresse wurden mit einem Preßdruck von 65 bar 4mm Stränge erzeugt, 12 h bei 120°C getrocknet und 12 h bei 500°C kalziniert.

BET: 250 m²/g

### Katalysator K

50 g Katalysator D wurden auf einer Fritte in ein beheiztes Glasrohr eingebaut und mit 2 l einer 20 %igen NH₄Cl-Lösung bei 80°C für 3 h im Kreislauf überspült. Anschließend wurde die NH₄Cl-Lösung erneuert und der Vorgang wiederholt. Der Katalysator wurde anschließend im geraden Durchgang mit Wasser gewaschen, 2 h bei 110°C getrocknet und 3 h bei 300°C kalziniert.

BET: 193 m²g⁻¹

### Katalysator L

1,1 l einer 0,33 m ZrOCl₂-Lösung wurden 24 h unter Rückfluß gekocht. Nach Verdünnung mit dest. H₂O auf 4,5 l wurden bei 20 bis 25°C 90 g Terrana D hinzugefügt und 1 h gerührt. Das Pulver wurde abfiltriert, zweimal mit je 1,5 l heißem dest. H₂O gewaschen, bei 110°C 2 h getrocknet und bei 250°C 3 h kalziniert.

### Katalysator M

98 g Katalysator L wurden mit 24,5 g Boehmit und 2,5 g Ameisensäure versetzt. Im Kneter wurde die Mischung kompaktiert und unter vorsichtigem Wasserzusatz (50 ml) verknetet. In einer Strangpresse wurden mit einem Preßdruck von 90 bar 1,5 mm Stränge erzeugt, 16 h bei 120°C getrocknet und 5 h bei 300°C kalziniert.

### Aminierungsbeispiele

Die Versuche wurden in einem Rohrreaktor (6 mm Innendurchmesser) unter isothermen Bedingungen bei 270°C bis 300C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse mit den verschiedenen Katalysatoren sind in der Tabelle 1 zusammengestellt.

**Tabelle 1**

| tert.-Butylamin (NH₃: C₄H₈ = 1,5) | | | | | | | |
|---|---|---|---|---|---|---|---|
| Kat. | Anteil Verstrangungshilfsmittel | Druck | Temp. | tert.-Butylamin-Ausbeute [Gew.-%] | | | Litergewicht |
| | Al₂O₃ _{[%]} | [bar] | [°C] | WHSV 0,7 [g/g·h] | WHSV 1,5 [g/g·h] | WHSV 3 [g/g·h] | [kg/l] |
| B | 40 | 280 | 300 | 13,26 | 10,38 | 7,72 | 0,7 |
| D | 40 | 280 | 300 | 11,33 | 5,91 | 2,86 | 0,71 |
| F | 20 | 280 | 300 | 13,25 | 9,04 | 5,65 | 0,80 |
| H | 20 | 280 | 300 | 13,16 | 10,80 | | |
| J | 20 | 280 | 300 | 14,01 | 11,34 | 7,20 | 0,62 |
| K | 40 | 280 | 300 | 12,52 | 7,95 | 5,79 | 0,7 |
| M | 20 | 280 | 300 | 12,95 | 9,89 | 6,96 | 0,96 |

## Patentansprüche

1. Verfahren zur Herstellung von Aminen der allgemeinen Formel I 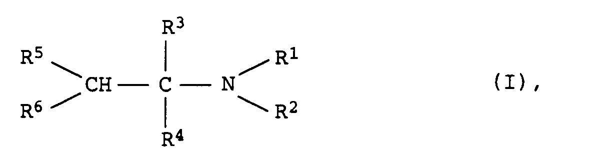 in der
R¹,R²,R³,R⁴,R⁵,R⁶ Wasserstoff, C₁- bis C₂₀-Alkyl, C₂- bis C₂₀-Alkenyl, C₂-bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
R¹ und R² gemeinsam eine gesättigte oder ungesättigte C₃- bis C₉-Alkylendikette und
R³ oder R⁵ C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 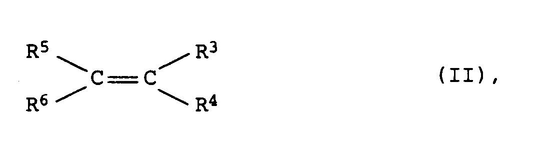 in der R³, R⁴, R⁵ und R⁶ die oben genannten Bedeutungen haben, mit Ammoniak oder primären oder sekundären Aminen der allgemeinen Formel III 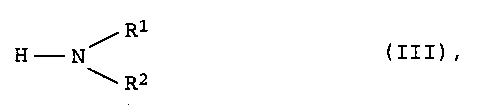 in der R¹ und R² die oben genannten Bedeutungen haben, bei Temperaturen von 200 bis 350°C und Drücken von 100 bis 300 bar in Gegenwart eines Heterogenkatalysators, dadurch gekennzeichnet, daß man als Heterogenkatalysator Pillared (Interlayered) Clays einsetzt.

2. Verfahren zur Herstellung von Aminen I nach Anspruch 1, dadurch gekennzeichnet, daß man das gebildete Amin I abtrennt und die nichtumgesetzten Einsatzstoffe II und III zurückführt.

3. Verfahren zur Herstellung von Aminen nach den Ansprüchen 1 bis 2, dadurch gekennzeichnet, daß man als Olefin II Isobuten, Diisobuten, Cyclopenten, Cyclohexen oder Polyisobuten einsetzt.

4. Verfahren zur Herstellung von Aminen nach den Ansprüchen 1 bis 3, dadurch gekennzeichnet, daß man als Heterogenkatalysatoren Aluminium-, Zirkon-, Titan-, Chrom-, Gallium-, Eisen-, Tantal- oder Silizium-Pillared Clays einsetzt.

5. Verfahren zur Herstellung von Aminen nach den Ansprüchen 1 bis 4, dadurch gekennzeichnet, daß man als Heterogenkatalysatoren Pillared Clays, die mit einer Säure, insbesondere einer aus der Gruppe Salzsäure, Flußsäure, Schwefelsäure, Phosphorsäure, Oxalsäure oder deren Gemische behandelt sind, einsetzt.

6. Verfahren zur Herstellung von Aminen nach den Ansprüchen 1 bis 5, dadurch gekennzeichnet, daß man als Heterogenkatalysatoren Pillared Clays, die mit einem oder mehreren Übergangsmetallen dotiert sind, einsetzt.

7. Verfahren zur Herstellung von Aminen nach den Ansprüchen 1 bis 6, dadurch gekennzeichnet, daß man als Heterogenkatalysatoren Pillared Clays, die mit einem oder mehreren Elementen der Seltenen Erden dotiert sind, einsetzt.

8. Verfahren zur Herstellung von Aminen nach den Ansprüchen 1 bis 7, dadurch gekennzeichnet, daß man als Heterogenkatalysatoren Pillared Clays in der Ammoniumform einsetzt.

9. Verfahren zur Herstellung von Aminen nach den Ansprüchen 1 bis 8, dadurch gekennzeichnet, daß man als Heterogenkatalysatoren Pillared Clays einsetzt, die mit einem Bindemittel verformt und bei Temperaturen von 200 bis 600°C calciniert sind.
